# EUROPÄISCHE PATENTSCHRIFT

(11) **EP 3 034 124 B1**
(45) Veröffentlichungstag und Bekanntmachung des Hinweises auf die Patenterteilung: **21.08.2019**
(21) Anmeldenummer: 15198065.3
(22) Anmeldetag: 04.12.2015
(51) Int. Cl.: A61M 25/00

(54) **SCHLAUCHSYSTEM FÜR DIE MEDIZINISCHE ANWENDUNG**
HOSE SYSTEM FOR MEDICAL USE
SYSTEME DE TUYAU POUR L'APPLICATION MEDICALE

(30) Priorität: 15.12.2014 DE 102014225939
(43) Veröffentlichungstag der Anmeldung: 22.06.2016
(73) Patentinhaber: RAUMEDIC AG, 95213 Münchberg (DE)
(72) Erfinder: Gläsel, Björn, 95158 Kirchenlamitz (DE); Wunderlich, Axel, 08626 Ebmath (DE)
(74) Vertreter: Rau, Schneck & Hübner Patentanwälte Rechtsanwälte PartGmbB

(56) Entgegenhaltungen:
- WO-A2-2010/075565
- DE-A1- 3 107 392
- DE-A1-102009 025 347
- US-A- 5 807 331
- US-A- 5 823 995
- US-A1- 2002 177 841
- US-B1- 6 544 215

## Beschreibung

Die Erfindung betrifft ein Schlauchsystem für die medizinische Anwendung.

Ein derartiges Schlauchsystem ist bekannt aus der US 5,807,331, die ein Schlauchsystem gemäß dem Oberbegriff des Anspruchs 1 offenbart, der US 6,544,215 B1, der DE 10 2009 025 347 A1, der US 5,823,995, der US 2002/0177841 A1, der DE 31 07 392 A1 und der US 2003/0135156 A1. Es ist eine Aufgabe der vorliegenden Erfindung, die Handhabbarkeit eines derartigen Schlauchsystems zu verbessern.

Die Aufgabe ist erfindungsgemäß gelöst durch ein Schlauchsystem mit den im Anspruch 1 angegebenen Merkmalen.

Erfindungsgemäß wurde erkannt, dass die Klebstoffschicht zwischen der Außenwand des eingesetzten Führungsdrahtes und der Innenwand des Führungsdraht-Schlauchlumens für eine Fixierung des Führungsdrahtes im Führungsdraht-Schlauchlumen und damit für eine Verdrehsicherung des Führungsdrahtes relativ zum Schlauch sorgt. Dies verbessert die Handhabung des Schlauchsystems, da insbesondere bei Ausübung einer Torsionskraft der Schlauch nicht über eine Relativverdrehung relativ zum inneren Führungsdraht einer solchen Torsionskraft unerwünscht ausweicht. Eine Querschnittsprofilierung des Führungsdrahtes bzw. des Führungsdraht-Schlauchlumens zur Sicherstellung einer Verdrehsicherung ist dann nicht erforderlich. Entsprechend kann der Führungsdraht einen runden Außenquerschnitt und das Führungsdraht-Schlauchlumen kann einen runden Innenquerschnitt aufweisen. Alternativ ist zur zusätzlichen Verdrehsicherung eine entsprechende, nicht rotationssymmetrische Profilierung der Außenwand des Führungsdrahtes bzw. der Innenwand des Führungsdraht-Schlauchlumens möglich. Der Führungsdraht sorgt innerhalb des Schlauchsystems für eine Versteifung des Schlauchs und damit für eine Führungsverbesserung. Der Führungsdraht kann elastisch und/oder plastisch verformbar sein.

Die Klebstoffschicht kann insbesondere den Führungsdraht auch stirnseitig abdecken. Diese Ummantelung kann auch im Bereich der stirnseitigen Enden des Führungsdrahtes vorliegen. Hierüber ist eine dichte Versiegelung des Führungsdrahtes nach außen möglich. Es ergibt sich insbesondere ein Korrosionsschutz des Führungsdrahtes.

Ein Kupferdraht nach Anspruch 2 kann insbesondere versilbert ausgeführt sein. Eine derartige Ausführung des Führungsdrahtes hat sich in der Praxis bewährt.

Ein gesteuert aushärtbarer Klebstoff nach Anspruch 3 sorgt für definierte Bedingungen bei der Erzeugung der Klebstoffschicht. Vor dem Aushärten kann der Klebstoff insbesondere als niederviskose Flüssigkeit vorliegen. Bei dem Klebstoff kann es sich um einen UV-aushärtbaren Kleber, z.B. auf Cyanacrylat-Basis handeln. Alternativ oder zusätzlich kann es sich um einen Klebstoff handeln, der unter Einfluss von Luftfeuchtigkeit und/oder Temperatur aushärtet. Auch ein Kleber mit langer Aushärtzeit ist zur Ausbildung der Klebstoffschicht möglich.

Ein Mehrlumenschlauch nach Anspruch 4 vergrößert die Einsatzmöglichkeiten des Schlauchsystems. In genau ein Lumen oder in mehrere Lumen eines solchen Mehrlumenschlauchs kann der Führungsdraht eingesetzt sein. Insbesondere ist eine Medien-Zudosierung oder auch eine Probenentnahme über den Schlauch des Schlauchsystems möglich.

Ein Drei-Lumenschlauch nach Anspruch 5 kann insbesondere als Dosiersystem für einen Zwei-Komponenten-Gewebekleber zum Einsatz kommen.

Ein Vier-Lumenschlauch nach Anspruch 6 kann insbesondere als Dosiersystem zum Auftrag einer Schutzschicht auf Gewebematerial zum Einsatz kommen. Eine derartige Schutzschicht kann ein unerwünschtes Verkleben des Gewebematerials verhindern. Zwei der vier Lumen können dann zur Zufuhr eines Zwei-K-Materials dienen. Ein weiteres Lumen kann zur Zufuhr eines Verwirbelungsgases dienen.

Der Mehrlumenschlauch kann auch mehr als vier Lumen aufweisen.

Ausführungsbeispiele der Erfindung werden nachfolgend anhand der Zeichnung näher erläutert. In dieser zeigen:
Fig. 1 schematisch ein Schlauchsystem, ausgebildet als Mehrlumen-Schlauchsystem, für die medizinische Anwendung in einer Seitenansicht;
Fig. 2 in einem Längsschnitt ein Führungsdraht-Schlauchlumen eines Mehrlumenschlauchs des Mehrlumen-Schlauchsystems nach Fig. 1;
Fig. 3 einen Schnitt durch den Mehrlumenschlauch gemäß Linie III-III in Fig. 1; und
Fig. 4 bis 6 jeweils in einer zu Fig. 3 ähnlichen Darstellung weitere Ausführungen von Mehrlumenschläuchen zum Einsatz in einem Mehrlumen-Schlauchsystem nach Art der Fig. 1 und 2.

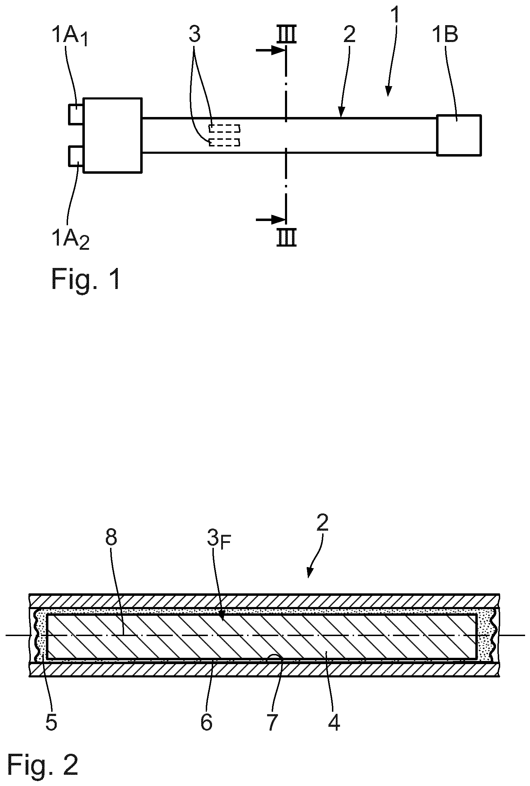

Ein in der Fig. 1 schematisch dargestelltes Schlauchsystem 1 kommt beispielsweise bei der Verarbeitung von Mehrkomponeten-Gewebeklebern bei medizinischen Eingriffen zum Einsatz.

Das Schlauchsystem 1 hat einen Schlauch 2 mit einer Mehrzahl von Schlauchlumen 3, von denen in der Fig. 1 zwei Schlauchlumen 3 gestrichelt angedeutet sind. Der Schlauch 2 wird nachfolgend auch als Mehrlumenschlauch bezeichnet. Die Schlauchlumen 3 können einen Innendurchmesser im Bereich zwischen 0,5 und 5 mm haben.

Der Schlauch 2 ist aus Kunststoff. Der Schlauch 2 kann insbesondere aus Polyvinylchlorid (PVC) oder Silikon gefertigt sein. Der Schlauch 2 kann auch aus einem Thermoplast gefertigt sein.

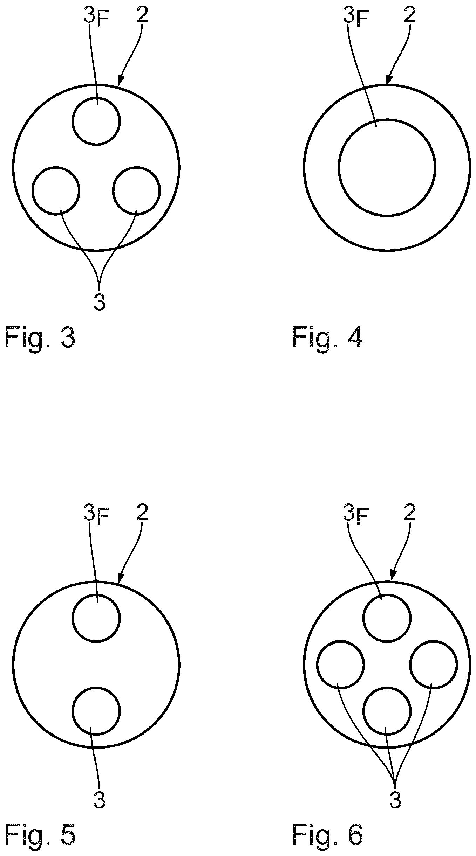

Fig. 3 zeigt einen Querschnitt durch den Schlauch 2. Der Schlauch 2 hat insgesamt drei Schlauchlumen 3, von denen eines als Führungsdraht-Schlauchlumen genutzt wird und nachfolgend auch die Bezugsziffer 3_{F} erhält.

Fig. 2 zeigt einen Längsschnitt eines Abschnittes des Schlauchs 2 im Bereich des Führungsdraht-Schlauchlumens 3_{F}. Ein Führungsdraht 4, der auch als Mandrin bezeichnet ist, ist in einem Schlauchabschnitt in das Führungsdraht-Schlauchlumen 3_{F} eingesetzt. Eine Klebstoffschicht 5 ist zwischen einer Außenwand 6 des eingesetzten Führungsdrahts und einer Innenwand 7 des Führungsdraht-Schlauchlumens 3_{F} angeordnet.

Die Klebstoffschicht 5 sorgt für eine Fixierung des Führungsdrahtes 4 im Führungsdraht-Schlauchlumen 3_{F} und damit für eine Verdrehsicherung des Führungsdrahtes 4 im Schlauch 2. Das Material des Schlauchs 2 ist zum Material der Klebstoffschicht 5 kompatibel, sodass der Schlauch 2 mit der Klebstoffschicht 5 verklebbar ist. Eine entsprechende Kompatibilität gilt für das Material des Führungsdrahtes 4.

Der Führungsdraht 4 ist plastisch verformbar. Der Führungsdraht 4 ist als versilberter Kupferdraht ausgeführt. Der Führungsdraht 4 sorgt für eine Versteifung des Schlauchs 2 und damit für eine Führungsverbesserung. Eine Länge des Führungsdrahts 4 liegt im Bereich zwischen 50 mm und 200 mm. Es kann auch ein Führungsdraht hier mit einer Länge von bis zu 600 mm oder ein noch längerer Führungsdraht zum Einsatz kommen. Aufgrund der Verdrehsicherung kann der Schlauch 2 einer Torsionskraftbeaufschlagung insbesondere durch einen Operateur nicht durch eine Relativverdrehung des Führungsdraht-Schlauchlumens 3_{F} zum Führungsdraht 4 in Bezug auf eine Längsachse 8 des Führungsdraht-Schlauchlumens 3_{F} ausweichen.

Die Klebstoffschicht 5 ummantelt den Führungsdraht 4 vollständig, also auch im Bereich von dessen stirnseitigen Enden. Hierdurch wird eine dichte Versiegelung des Führungsdrahts 4 nach außen und damit ein effizienter Korrosionsschutz des Führungsdrahtes 4 erreicht.

Die Klebstoffschicht 5 ist aus einem gesteuert aushärtbaren Klebstoff gebildet. Bei dem beschriebenen Ausführungsbeispiel handelt es sich bei dem Klebstoff der Klebstoffschicht 5 um einen UV-aushärtbaren Kleber auf Cyanacrylat-Basis. Auch ein anderer Klebstoff, der gezielt aushärtbar ist und beispielsweise unter dem Einfluss von Luftfeuchtigkeit und/oder Temperatur aushärtet, oder ein Kleber mit langer Aushärtezeit, kann für die Klebstoffschicht 5 zum Einsatz kommen.

Das Schlauchsystem 1 mit dem Schlauch 2 kann als Dosiersystem für einen Zwei-Komponenten-Gewebekleber zum Einsatz kommen. Über die beiden Schlauchlumen 3, in die der Führungsdraht 4 nicht eingesetzt ist, werden die beiden Komponenten des Zwei-Komponenten-Gewebeklebers einem Einsatzort zugeführt. Je nach Anzahl der Schlauchlumen 3, die auch größer sein kann als zwei, kann eine entsprechende Anzahl von Medien längst des Schlauchs 2 geführt werden.

Das Schlauchsystem 1 und insbesondere der Schlauch 2 mit dem Führungsdraht 4 wird folgendermaßen hergestellt: Zunächst wird der Führungsdraht 4 und der Schlauch 2 auf eine Fixlänge geschnitten. Der Schlauch 2 wird dabei auf Überlänge geschnitten. Anschließend wird der Führungsdraht 4 in das Führungsdraht-Schlauchlumen 3_{F} eingeführt und in einer gewünschten Axialposition im Führungsdraht-Schlauchlumen 3_{F} fixiert. Anschließend wird das Führungsdraht-Schlauchlumen 3_{F} im Bereich des Führungsdrahtes 4 vollständig mit dem zu diesem Zeitpunkt niederviskos flüssigen Klebstoff vollständig gefüllt. Dies erfolgt unter Nutzung des Kapillareffektes. Alternativ oder zusätzlich kann zur Förderung einer Füllung des Führungsdraht-Schlauchlumens 3_{F} im Bereich des Führungsdrahts 4 mit dem Klebstoff diesem Lumen Unterdruck zugeführt werden. Der niederviskose Klebstoff wird dabei von einer Klebstoff-Auftragsseite her axial durch den gesamten Zwischenraum zwischen der Außenwand 6 des eingesetzten Führungsdrahtes 4 und der Innenwand 7 des Führungsdraht-Schlauchlumens 3_{F} bis zur der Klebstoff-Aufbringseite gegenüberliegenden Stirnseite des Führungsdrahtes 4 transportiert. Hierbei bilden sich stirnseitige Klebstoffschichten, die Stirnseiten des Führungsdrahtes 4 bedecken (vgl. Fig. 2), so dass der Führungsdraht 4 vollständig vom Klebstoff umgeben ist. Anschließend wird der Klebstoff zur Klebstoffschicht 5 ausgehärtet. Nicht vergossene Enden des Führungsdraht-Schlauchlumens 3_{F} können dann abgeschnitten werden.

Zum Schlauchsystem 1 nach Fig. 1 gehören neben dem Schlauch 2 noch ein Medien-Zuführstutzen 1A mit zwei Zuführ-Ports 1A₁ und 1A₂. Ferner gehört zum Schlauchsystem 1 noch ein Mischteil 1B zum Vermischen von über Schlauchlumen 3 zugeführten Medien. Das Mischteil 1B kann als Düse ausgeführt sein. Über die Schlauchlumen 3 können verschiedene Medien geführt werden; es kann über die Schlauchlumen 3 aber auch ein und dasselbe Medium geführt werden.

Die Fig. 4 bis 6 zeigen weitere Varianten von Schläuchen 2, die anstelle des Mehrlumenschlauchs nach Fig. 3 beim Schlauchsystem 1 zum Einsatz kommen können.

Der Schlauch 2 nach Fig. 4 hat ein einziges Schlauchlumen 3, das gleichzeitig das Führungsdraht-Schlauchlumen 3_{F} darstellt. Beim Verwenden eines Ein-Lumenschlauchs 2 nach Fig. 4 kann ein Zuführstutzen nach Art des Stutzens 1A und auch ein Mischteil nach Art des Mischteils 1B für das Schlauchsystem 1 entfallen.

Der Schlauch 2 nach Fig. 5 hat zwei Schlauchlumen 3, von denen eines das Führungsdraht-Schlauchlumen 3_{F} darstellt. Über das andere Schlauchlumen 3 kann dann ein Medium zu- oder abgeführt werden.

Der Schlauch 2 nach Fig. 6 ist als Vier-Lumen-Schlauch ausgeführt, wobei eines dieser vier Schlauchlumen 3 wiederum das Führungsdraht-Schlauchlumen 3_{F} darstellt. Zwei der drei nicht als Führungsdraht-Schlauchlumen 3_{F} genutzten Schlauchlumen 3 des Vier-Lumen-Schlauchs 2 können wiederum als Transport eines Zwei-Komponenten-Materials genutzt werden. Das dann noch verbleibende vierte Schlauchlumen 3 kann zum Transport eines Verwirbelungsgases eingesetzt werden. Der so genutzte Vier-Lumen-Schlauch 2 kann in einem Dosiersystem zum Auftrag einer Schutzschicht auf Gewebematerial genutzt werden, wobei eine derartige Schutzschicht ein unerwünschtes Verkleben von Gewebekomponenten verhindert.

## Patentansprüche

1. Schlauchsystem (1) für die medizinische Anwendung
- mit einem Schlauch (2) mit mindestens einem Schlauchlumen (3_{F}),
- mit einem Führungsdraht (4) der zumindest in einem Schlauchabschnitt im mindestens einen Schlauchlumen (3_{F}) eingesetzt ist,
- mit einer Klebstoffschicht (5) zwischen einer Außenwand (6) des eingesetzten Führungsdrahts (4) und einer Innenwand (7) des Führungsdraht-Schlauchlumens (3_{F}),
**dadurch gekennzeichnet,**
- **dass** die Klebstoffschicht (5) so ausgeführt ist, dass sie für eine Verdrehsicherung des Führungsdrahtes (4) relativ zum Schlauch (2) sorgt, und
- **dass** die Klebstoffschicht (5) den Führungsdraht (4) vollständig ummantelt.

2. Schlauchsystem nach Anspruch 1, **dadurch gekennzeichnet, dass** der Führungsdraht (4) als Kupferdraht ausgeführt ist.

3. Schlauchsystem nach einem der Ansprüche 1 oder 2, **dadurch gekennzeichnet, dass** die Klebstoffschicht (5) aus einem gesteuert aushärtbaren Klebstoff gebildet ist.

4. Schlauchsystem nach einem der Ansprüche 1 bis 3, **dadurch gekennzeichnet, dass** der Schlauch (2) als Mehrlumenschlauch ausgeführt ist.

5. Schlauchsystem nach Anspruch 4, **dadurch gekennzeichnet, dass** der Mehrlumenschlauch (2) als Drei-Lumenschlauch ausgeführt ist.

6. Schlauchsystem nach Anspruch 4, **dadurch gekennzeichnet, dass** der Mehrlumenschlauch (2) als Vier-Lumenschlauch ausgeführt ist.

## Claims

1. Tube system (1) for medical use
- having a tube (2) with at least one tube lumen (3_{F}),
- having a guide wire (4) which is inserted in the at least one tube lumen (3_{F}) at least in a tube portion,
- having an adhesive layer (5) between an outer wall (6) of the inserted guide wire (4) and an inner wall (7) of the guide wire tube lumen (3_{F}),
**characterized**
- **in that** the adhesive layer (5) is embodied such that it ensures prevention of rotation of the guide wire (4) relative to the tube (2), and
- **in that** the adhesive layer (5) completely covers the guide wire (4).

2. Tube system according to Claim 1, **characterized in that** the guide wire (4) is embodied as a copper wire.

3. Tube system according to either of Claims 1 and 2, **characterized in that** the adhesive layer (5) is formed from an adhesive which is able to be cured in a controlled manner.

4. Tube system according to one of Claims 1 to 3, **characterized in that** the tube (2) is embodied as a multi-lumen tube.

5. Tube system according to Claim 4, **characterized in that** the multi-lumen tube (2) is embodied as a three-lumen tube.

6. Tube system according to Claim 4, **characterized in that** the multi-lumen tube (2) is embodied as a four-lumen tube.

## Revendications

1. Tubulure (1) destinée à une application médicale, comprenant
- un tube (2) comportant au moins une lumière de tube (3_{F}),
- un fil de guidage (4) inséré dans au moins une section de tube dans l'au moins une lumière de tube (3_{F}),
- une couche adhésive (5) entre une paroi extérieure (6) du fil de guidage (4) inséré et une paroi intérieure (7) de la lumière de tube du fil de guidage (3_{F}),
**caractérisée en ce**
- **que** la couche adhésive (5) est conçue de manière à assurer une protection anti-rotation du fil de guidage (4) par rapport au tube (2), et en ce
- **que** la couche adhésive (5) enveloppe entièrement le fil de guidage (4).

2. Tubulure selon la revendication 1, **caractérisée en ce que** le fil de guidage (4) est conçu comme du fil de cuivre.

3. Tubulure selon l'une des revendications 1 ou 2, **caractérisée en ce que** la couche adhésive (5) est formée à partir d'un adhésif durcissable de manière commandée.

4. Tubulure selon l'une des revendications 1 à 3, **caractérisée en ce que** le tube (2) est conçu comme un tube à plusieurs lumières.

5. Tubulure selon la revendication 4, **caractérisée en ce que** le tube à plusieurs lumières (2) est conçu comme un tube à trois lumières.

6. Tubulure selon la revendication 4, **caractérisée en ce que** le tube à plusieurs lumières (2) est conçu comme un tube à quatre lumières.
